# FASCICULE DE BREVET EUROPEEN

(11) **EP 0 729 382 B1**
(45) Date de publication et mention de la délivrance du brevet: **12.05.1999**
(21) Numéro de dépôt: 95901495.2
(22) Date de dépôt: 16.11.1994
(51) Int. Cl.: B01F 17/00, C11D 1/66, C11D 3/20, A61K 7/50, A61K 7/48

(54) **UN CONCENTRE COMPRENANT DES ALKYLGLYCOSIDES ET SES UTILISATIONS**
ALKYLGLYKOSIDEN ENTHALTENDES KONZENTRAT UND SEINE ANWENDUNGEN
ALKYLGLYCOSIDE-CONTAINING CONCENTRATE AND USES THEREOF

(30) Priorité: 19.11.1993 FR 9313895
(43) Date de publication de la demande: 04.09.1996
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: AMALRIC, Chantal, F-91700 Blan (FR); LECOCU-MICHEL, Nelly, F-94700 Maison-Alfort (FR)
(74) Mandataire: Le Moenner, Gabriel
(86) Numéro de dépôt international: FR9401336
(87) Numéro de publication internationale: WO9513863

(56) Documents cités:
- EP-A- 0 474 915
- WO-A-92/06778
- WO-A-92/07543
- WO-A-94/29416

## Description

La présente invention concerne un concentré comprenant un mélange d'alkylglycosides et au moins un alcool gras, ainsi que des utilisations de ces concentrés, notamment en tant qu'agent nacrant ou pour préparer des émulsions.

Les alkylglycosides sont des composés bien connus.

Ils peuvent notamment être préparés selon les procédés décrits dans les demandes WO-A-94/20513 (publieé le 15.09.94) et FR-A-2 702 769 (publiee le 23.09.94), toutes deux au nom de la demanderesse.

Ils peuvent être utilisés comme tensio-actifs, seuls ou en association avec d'autres tensio-actifs, dans de nombreuses applications. A cet égard, on peut par exemple faire référence à la brochure Triton CG 110, de mai 1975, diffusée par la société Rohm & Haas ou à la demande de brevet EP-A-70.074, qui décrivent des compositions et formulations associant des tensio-actifs et des alkylglycosides, utiles notamment en détergence ou en cosmétique.

La demande de brevet WO 92/07543 décrit des émulsions comprenant des alkylglycosides C₁₂-C₁₈ comme émulsifiants, des alcools gras pour régler la consistance, ainsi qu'un inhibiteur de cristallisation.

La demande de brevet WO 92/06778, au nom de la demanderesse, décrit des compositions comprenant 60 à 90 % en poids d'au moins un alcool gras ayant de 12 à 22 atomes de carbone et de 10 à 40 % en poids d'un alkylpolyoside (ou alkylglycoside) dont la partie alkyle peut être identique à celle de l'alcool gras.

Ces compositions sont utiles en tant qu'agents autoémulsionnables.

Un premier objet de la présente invention consiste en une composition sous forme d'un concentré à base d'alkylglycosides présentant des propriétés, qui, à la connaissance de la demanderesse, n'ont jamais été obtenues ou atteintes auparavant au moyen d'alkylglycosides.

Un second objet de la présente invention consiste en l'utilisation dudit concentré en tant qu'agent nacrant, en vue de produire un aspect nacré, stable dans le temps.

Un troisième objet de la présente invention consiste en l'utilisation dudit concentré pour la préparation d'émulsion, en particulier d'émulsions fluides, tels des laits, stables dans le temps.

Un quatrième objet de l'invention consiste en des compositions, notamment des syndets ou des savons de toilette, comportant ledit concentré, peu ou pas irritante pour l'homme ou l'animal.

Un cinquième objet de l'invention consiste en des compositions moussantes comprenant une phase huile émulsionnée, stable dans le temps.

Selon un premier aspect de l'invention, celle-ci consiste en un concentré tel que défini dans la revendication 1,
ii) de 10 à 40 % en poids d'au moins un alcool gras de formule R₁OH, R₁ étant un radical aliphatique, linéaire ou ramifié, saturé ou insaturé, comportant de 8 à 22 atomes de carbone.

Le concentré selon l'invention peut notamment être utilisé comme agent nacrant pour procurer un effet nacrant homogène et stable dans le temps. Ceci est tout à fait surprenant dans la mesure où des compositions telles que celles décrites dans la demande de brevet WO 92/06778, mentionnée plus haut, ne peuvent conférer un tel effet nacrant.

Les alkylglycosides de formules (I) et (II) peuvent comporter, à titre de reste d'un saccharide G ou G', un reste glucose, mannose, galactose, idose, arabinose, xylose, ribose, gulose, lyxose ou fructose. Avantageusement, G et G' sont identiques et représentent chacun un reste glucose. G et G' peuvent être sous forme anomérique α ou β, et le motif saccharide sous forme furanoside ou pyranoside.

Les indices x et x' représentent le degré de Polymérisation moyen du reste saccharide. Ils représentent de préférence un nombre compris entre 1,05 et 2,5, plus préférentiellement compris entre 1,05 et 1,35.

On note que lorsque l'un au moins de x et x' est égal à 1, l'alkylglycoside correspondant peut être qualifié d'alkylmonoglycoside. Quand l'un de x ou de x' est supérieur à 1, l'alkylglycoside correspondant peut être qualifié d'alkylpolyglycoside.

Les alkylglycosides de formule (I) ont un radical R pouvant de préférence comporter de 10 à moins de 16 atomes de carbone, plus préférentiellement de 12 à moins de 16 atomes de carbone.

Les alkylglycosides de formule (II) constitutifs du concentré, sont tout particulièrement ceux pour lesquels R' est un radical comportant 16 et/ou 18 atomes de carbone.

Les radicaux R, R' et R₁ sont de manière avantageuse des radicaux aliphatiques saturés, c'est-à-dire des radicaux alkyles.

Le concentré selon l'invention comprend habituellement un mélange d'au moins deux alcools gras. Le radical R₁ de chacun de ces alcools gras est préférentiellement identique à chacun des radicaux R et R'. Ledit mélange d'alcool gras est alors constitué d'une part, d'au moins un alcool gras comportant de 8 à moins de 16 atomes de carbone, de préférence de 10 à moins de 16 atomes de carbone, plus préfentiellement de 12 à moins de 16 atomes de carbone, et, d'autre part, d'au moins un alcool gras comportant de 16 à 22 atomes de carbone, de préférence de 16 à 18 atomes de carbone. Ce mélange d'alcools gras comporte plus particulièrement au moins 80 % en poids, de préférence de 90 à 99 % en poids d'un ou plusieurs alcools gras comportant 16 ou plus atomes de carbone.

Un concentré selon l'invention, comprend habituellement de 15 à 38 %, de préférence de 25 à 38 % en poids d'au moins un alcool gras.

Outre les alkylglycosides et alcools gras mentionnés ci-dessus, ledit concentré peut comporter un saccharide et/ou un polysaccharide en une concentration inférieure ou égale à 5% en poids.

Les concentrés selon l'invention peuvent être préparés par simple mélange de leurs constituants en des proportions telles que mentionnées ci-dessus.

Mais à l'échelle industrielle, on les prépare de préférence selon l'une des deux voies classiquement utilisées pour la synthèse des alkylglycosides.

La première voie comprend la réaction, en milieu acide, entre un alcool gras et un saccharide disposant d'un OH anomérique, tel le glucose ou le dextrose.

La deuxième voie consiste :
- dans une première étape à préparer un premier alkylpolyglycoside en procédant à une réaction d'étherification entre un alcool léger comme par exemple, le méthanol ou le butanol et un saccharide tel le glucose, pour obtenir des composés tels le méthylglucoside ou le butylglucoside; puis,
- dans une seconde étape, à effectuer une transétherification avec un alcool gras lourd tels ceux mentionnés ci-dessus, avec distillation de l'alcool léger.

Chacune de ces deux voies peut être, le cas échéant, complétée par des opérations de neutralisation, de filtration, de décoloration et d'élimination de l'alcool gras en excès. Cette élimination peut se faire par distillation, notamment au moyen d'un évaporateur à film mince, de sorte à permettre l'obtention d'une concentration en alcool gras telle que spécifiée plus haut.

Habituellement, les concentrés ainsi préparés sont solides à la température ambiante. Ils peuvent se présenter sous forme d'écaille.

Selon un autre aspect de l'invention, celle-ci concerne l'utilisation des concentrés décrits ci-dessus pour diverses applications. Ainsi lesdits concentrés peuvent entrer dans la formulation de diverses compositions, qui sont décrites ci-dessous.

L'une des utilisations principales des concentrés de l'invention est, comme indiqué plus haut, celle d'agent nacrant. L'aspect nacré produit par ledit concentré est habituellement obtenu par différents types de cristaux solides, organiques ou inorganiques, comme les esters d'acides gras, le mica ou l'oxychlorure de bismuth. Sans être tenu par la théorie, il semble que le concentré selon l'invention procure un aspect nacré par formation de gouttelettes ou de cristaux liquides réfléchissant la lumière. Par le terme "agent nacrant", on entend également ici un agent produisant un aspect ou un effet irisé, moiré ou métallisé.

Il est particulièrement notable que l'aspect nacré produit par un concentré selon l'invention est homogène et stable dans le temps.

Les compositions pouvant comporter à titre d'agent nacrant, le concentré selon l'invention peuvent être choisies parmi les compositions moussantes suivantes : les compositions pharmaceutiques, cosmétiques ou hygiéniques, tels les shampooings, les savons liquides, les gels douches, les bains moussants, les gels démaquillant visage ou des compositions détergentes, notamment les détergents vaisselles et les lessives liquides. Dans ce type de composition, le concentré selon l'invention peut avoir un rôle de tensio-actif moussant.

La concentration dans une telle composition en concentré selon l'invention, peut être comprise entre 2 et 15 % en poids.

Les compositions mentionnées peuvent comporter en outre des tensio-actifs, notamment des tensio-actifs moussants. A titre de tensio-actifs, on peut citer :
- les tensio-actifs amphotères, notamment la bétaïne et ses dérivés tels les alkylbétaïnes, comme les N-alkylbétaïnes ou les C-alkylbétaïnes, les N-alkylaminobétaïnes, les alkylamidobétaines, les alkylphosphoamidobétaïnes ou les alkylphosphobétaïnes, la sultaïne et ses dérivéz, tels les alkylamidosulfobétaïnes, les dérivés de l'imidazoline, les N-alkylglycines, les N-alkyl-β-alanine, les alkylpolyaminecarboxylates et les N-alkylaminobutyrates.

Des tensio-actifs amphotères préférés sont choisis parmi la cocamidobétaïne, la cocobétaïne, la cocoamidosultaïne, la cocoamidopropylbétaïne, la stéarylbétaïne, la stéarylamphocarboxylique glycinate, la cocoamidopropylhydroxysulfobétaïne, la cocoamphocarboxyglycinate, la cocoimidazoline carboxylate ou la cocoamidopropyl diméthylamino hydroxy propyle d'hydrolysat de Collagène (selon nomenclature CFTA-Cosmetic Toiletries Fragrance Association).
- les tensio-actifs non ioniques, notamment les amides de coprah, les polysorbates, les huiles de ricin hydrogénées et polyoxyéthylénées (OE) et/ou polyoxypropylénées (OP), les alkylphénols OE et/ou OP ou les alkylglycosides tels ceux de formule (I) et (II) mentionnés ci-dessus.
- les tensio-actifs cationiques, notamment ceux du type ammonium quaternaire ou les oxydes d'amine.
- et, de manière avantageuse, les tensio-actifs anioniques, notamment ceux cités dans la demande de brevet EP-A-70.074 comme les carboxylates, les sulfonates et surtout, les sulfates. Parmi ces derniers, on préfère les alkylsulfates et les alkyléther sulfates neutralisés par un cation, tels les cations sodium, potassium, magnésium ou ammonium. Les lauryléther sulfates de sodium sont des tensio-actifs tout particulièrement préférés.

Par ailleurs, lesdites compositions moussantes peuvent comporter une phase huile émulsionnée. Généralement, ces compositions comportent ladite phase huile et l'un au moins des tensio-actifs mentionnés plus haut. De telles compositions peuvent consister en des shampooings, des liquides vaisselles et , tout particulièrement, des gels douches 2 en 1, c'est-à-dire des gels douche présentant simultanément un pouvoir nettoyant et hydratant.

Les compositions classiques, comportant une phase huile et au moins un tensio-actif moussant sont habituellement instables dans le temps, c'est-à-dire que ladite phase huile déphase.

Pour remédier à cette instabilité, on y incorpore un agent stabilisant tel des polymères épaississants. Or, de manière surprenante, il a pu être constaté que la présence dans ces compositions du concentré selon l'invention, permet de conférer une grande stabilité dans le temps à la phase huile, même en l'absence de ces agents stabilisants.

Ces compositions comportent généralement, en poids, de 2 à 15 % dudit concentré, de 2 à 50 %, de préférence de 5 à 30 % d'au moins un tensio-actif, de 0,5 à 15 % d'huile et une phase aqueuse.

L'huile peut être d'origine minérale, végétale, animale ou synthétique. On peut notamment citer les huiles de silicone comme la diméthicone, l'huile de monoï, l'huile d'amande douce, l'huile de paraffine et les triglycérides synthétiques.

Selon un autre aspect, l'invention concerne l'utilisation dudit concentré pour la préparation d'émulsions, celles-ci peuvent être du type eau dans huile, huile dans eau ou huile dans eau dans huile.

Ledit concentré convient tout particulièrement pour la préparation d'émulsions fluides, stables dans le temps.

On note qu'il est tout à fait surprenant que de tels émulsions fluides, pouvant rester stables plus de 3 mois, puissent être préparées avec un concentré selon l'invention, dans la mesure où ni les alkylglycosides, ni les alcools n'en permettent l'obtention par ailleurs.

Dans le cadre de l'invention, on entend par "émulsion fluide" une émulsion dont l'écoulement au travers d'une coupe d'écoulement ISO 2431 de 6 mm commence moins de 5 secondes après l'enlèvement de l'obturateur (test selon la norme internationale ISO 2431).

A titre d'émulsions fluides, on peut notamment citer les laits, en particulier des laits du type huile dans eau, à usage cosmétiques ou hygiénique comme les laits démaquillants, les laits corporels ou les laits solaires.

Lesdites émulsions peuvent comporter de 1 à 15 % en poids en ledit concentré. La phase huile de ces émulsions peut être constituée d'une ou de plusieurs huiles, telles celles mentionnées ci-dessus.

Les compositions et émulsions décrites ci-dessus peuvent être préparées de manière connue de l'homme du métier, par exemple par simple mélange de leurs constituants. Le concentré selon l'invention peut être mis en oeuvre tel quel, ou, le plus suivant, à l'état fondu.

Il est généralement possible de le mettre en oeuvre sous la forme d'une base nacrante consistant en une dispersion dudit concentré dans un liquide approprié. Un tel liquide est habituellement l'eau, mais il peut également consisté en un solvant organique.

La concentration dudit concentré dans une base nacrante peut être comprise entre 1 et 50 % en poids, de préférence entre 2 et 25 % en poids.

Une telle base nacrante constitue un autre aspect de l'invention, de même que son utilisation comme agent nacrant et pour préparer des émulsions.

Selon un autre aspect, l'invention vise encore à couvrir des syndets, ou pains dermatologiques, comprenant un concentré selon l'invention, ainsi qu'un ou plusieurs tensio-actifs moussants tels ceux mentionnés plus haut, à l'exclusion des savons.

Habituellement, ces syndets comportent encore au moins un agent plastifiant, liant ou texturant classique, comme les polyéthylèneglycols (PEG), les maltodextrines ou les alcools gras.

Une syndet selon l'invention comprend généralement de 2 à 40 % en poids, de préférence de 10 à 35 % en poids dudit concentré, de 20 à 50 % en poids en au moins un tensio-actif moussant et de 10 à 40 % en poids d'au moins un agent plastifiant, liant ou texturant.

Les syndets selon l'invention, comportant ledit concentré, présentent une plasticité améliorée, un meilleur toucher de la mousse ainsi qu'une tolérance cutanée accrue. Elles peuvent être préparées de manière connue de l'homme du métier à partir du concentré à l'état fondu.

Selon un autre aspect, l'invention concerne aussi des savons de toilette comprenant ledit concentré défini plus haut, au moins un savon tensio-actif et, le cas échéant, au moins un agent adoucissant, tel la glycérine.

Ledit savon, tensio-actif peut consister en un acide gras, notamment un acide gras d'origine animale ou végétale, comme l'acide gras de suif ou de coprah, neutralisé par la soude.

Habituellement, un savon de toilette selon l'invention, comprend de 1 à 20 % en poids dudit concentré, de 65 à 95 % en poids, d'au moins un savon et, le cas échéant, de 3 à 15% en poids d'au moins un agent adoucissant. Il peut être préparé de manière connue de l'homme de métier à partir du concentré à l'état fondu.

Les compositions, émulsions, syndets et savons de toilettes mentionnés ci-dessus peuvent également comprendre d'autre additifs classiques comme des colorants, des parfums, des conservateurs ou des agents viscosants ou épaississants.

Les exemples qui suivent ont pour but d'illustrer la présente invention.

Sauf indication contraire, dans ces exemples, tous les % sont indiqués en poids.

Les composées ci-dessous sont mentionnés dans certains des exemples. Leur signification est la suivante :
Acrysol 22 : copolymère acrylate/steareth-20 methacrylate (Société Rohm & Haas),
Sepigel 305 : copolymère acrylique (société SEPPIC),
Oramix NS10 : Décylpolyglucoside (société SEPPIC),
LESNa : Lauryléthersulfate de sodium
Carbomer 941 : Polymère acrylique (Société GOODRICH)

### EXEMPLE 1

### Préparation d'un concentré comprenant des alkylglycosides

Dans un réacteur polyvalent on fait réagir, sous vide, à une température de 100°C environ et en présence d'un système catalytique constitué par 57g d'acide sulfurique et de 41g d'acide hypophosphoreux à 50 %, 6,30 kg de glucose anhydre et 51,5 kg d'alcool gras constitué (% en poids) de 20 % de dodécanol, de 28 % de tétradécanol, de 17 % d'hexadécanol et de 35 % d'octadécanol.

Après 5 heures la réaction est complétée et le mélange réactionnel est neutralisé par 0,1 kg de soude, filtré, et l'alcool gras en excès est partiellement éliminé par distillation sur un évaporateur à film mince pour conduire à 14kg d'un concentré comprenant (% en poids).

| | |
|---|---|
| • Dodécanol | 0,3 % |
| • Tétradécanol | 1,6 % |
| • Hexadécanol | 4,5 % |
| • Octadécanol | 27,9 % |
| • Dodécylpolyglucoside | 14,4 % |
| • Tétradécylpolyglucoside | 18,9 % |
| • Hexadécylpolyglucoside | 10,9 % |
| • Octadécyl polyglucoside | 21,5 % |

Les caractéristiques de ce concentré sont les suivantes:

| | |
|---|---|
| • indice d'acide | 0.4 |
| • indice d'hydroxyle | 445 |
| • pH (1%) | 5.05 |
| • couleur (Gardner) | 2 |
| • Point de fusion | 57°C |

### EXEMPLE 2

### Préparation d'un concentré comprenant des alkylglycosides

Dans les conditions de l'exemple 1, on fait réagir 6,45 kg de glucose et 50 kg d'alcools gras constitués (% en poids) de 10 % de décanol, 9 % de dodécanol, 21 % de tétradécanol, 32 % d'hexadécanol et de 28 % d'octadécanol.

Le concentré obtenu, comprend (% en poids)

| | |
|---|---|
| • Décanol | 0,1 % |
| • Dodécanol | 0,1 % |
| • Tétradécanol | 1,4 % |
| • Hexadécanol | 8,9 % |
| • Octadécanol | 19,9 % |
| • Décylpolyglucoside | 8,2 % |
| • Dodécylpolyglucoside | 6,8 % |
| • Tétradécylpolyglucoside | 14,9 % |
| • Hexadécylpolyglucoside | 21,6 % |
| • Octadécyl polyglucoside | 18,1 % |

Les caractéristiques de ce concentré sont les suivantes:

| | |
|---|---|
| • indice d'acide | 0,3 |
| • indice d'hydroxyle | 469 |
| • pH (1%) | 5.4 |
| • couleur (Gardner) | 2 |
| • Point de fusion | 58°C |

### EXEMPLE 3

### Préparation d'un concentré comprenant des alkylglycosides

Dans les conditions de l'exemple 1, on fait réagir 6,5 kg de glucose et 50 kg d'alcools gras constitués (% en poids) de 17 % de dodécanol, 22 % de tétradécanol, 39 % d'hexadécanol et 22 % d'octadécanol.

Le concentré obtenu, comprend (% en poids)

| | |
|---|---|
| • Dodécanol | 0,3 % |
| • Tétradécanol | 1,4 % |
| • Hexadécanol | 13,9 % |
| • Octadécanol | 20,2 % |
| • Dodécylpolyglucoside | 11,9 % |
| • Tétradécylpolyglucoside | 14,5 % |
| • Hexadécylpolyglucoside | 24,3 % |
| • Octadécylglucoside | 13,1 % |

Les caractéristiques de ce concentré sont les suivantes:

| | |
|---|---|
| • indice d'acide | 0,3 |
| • indice d'hydroxyle | 462 |
| • pH (1%) | 5.3 |
| • couleur (Gardner) | 3 |
| • Point de fusion | 57°C |

### EXEMPLE 4

On a préparé diverses dispersions dans l'eau de mélanges d'alkylpolyglucosides (APG) et d'alcools gras. Chacune de ces dispersions comportaient 15 % d'extrait sec.

La dispersion 1, selon l'invention, a été préparée à partir du concentré de l'exemple 2.

La dispersion 2, à titre comparatif, contenait un extrait sec de composition suivante :

| | |
|---|---|
| • Décylpolyglucoside | 28,2 % |
| • Dodécylpolyglucoside | 2,4 % |
| • Tétradécylpolyglucoside | 2,4 % |
| • Alcool cétyliques | 33,5 % |
| • Alcool stéarylique | 33,5 % |

La dispersion 3, à titre comparatif, contenait un extrait sec de composition suivante :

| | |
|---|---|
| • Hexadécylpolyglucoside | 25 % |
| • Octadécylpolyglucoside | 25 % |
| • Hexadécanol | 25 % |
| • Octadécanol | 25 % |

Les teneurs (en %) de l'extrait sec de chacune des dispersions 1 à 3, exprimées en APG et alcools gras ayant d'une part, une partie alkyle comportant moins de 16 atomes de carbone et, d'autre part, une partie alkyle comportant plus de 16 atomes de carbone, sont resumées dans le Tableau I ci-dessous.

**Tableau I**

| Dispersion | APG < C16 | APG ≥ C16 | Alcool < C16 | Alcool ≥ C16 |
|---|---|---|---|---|
| 1 | 29,9 | 39,7 | 1,6 | 28,8 |
| 2 | 33 | 0 | 0 | 67 |
| 3 | 0 | 50 | 0 | 50 |

A partir de chacune des dispersions 1 à 3, on a préparé des compositions moussantes 1, 2 et 3 comprenant :

| | |
|---|---|
| dispersion 1, 2 ou 3 | 20 % |
| LES Na | 12,5 % |
| Acrysol 22 | 3 % |
| eau | QSP 100 % |

On a alors examiné l'aspect et la stabilité de chacune des dispersions 1 à 3 et des compositions 1 à 3. Les résultats obtenus figurent dans le Tableau II.

**TABLEAU II**

| Produit testé | Aspect | Stabilité |
|---|---|---|
| Dispersion 1 | nacré, fluide et Hom | 1 mois |
| Dispersion 2 | non nacré et Het | 1 jour |
| Dispersion 3 | pateux, non nacré | 7 jours |
| Composition 1 | nacré et Hom | supérieure à 3 mois |
| Composition 2 | non nacré | 1 jour |
| Composition 3 | non nacré et Het | au moins 7 jours |
| Het : aspect hétérogène Hom : aspect homogène | | |

Les résultats du Tableau II montrent qu'une dispersion ou une composition comportant un concentré selon l'invention, présentent un aspect nacré, homogène et stable dans le temps.

### EXEMPLE 5

Afin d'apprécier la stabilité d'une composition comprenant une phase huile émulsionnée, on a préparé une composition, selon l'invention, du type gel douche 2 en 1, comprenant:

| | |
|---|---|
| Concentré de l'exemple 3 | 5 % |
| LES Na | 12,5 % |
| Huile | 1 ou 5 % |
| Acrysol 22 | 3 % |
| eau | QSP 100 % |
| soude | Q.S. pH = 7,2 |

A titre comparatif, on a préparé une composition témoin ne comportant pas le concentré de l'exemple 3.

La stabilité de chacune de ces compositions a été testée à 40°C. Les résultats obtenus figurent dans le Tableau III ci-dessous.

**TABLEAU II**

| **Huile** | **Composition témoin** | **Composition selon l'invention** |
|---|---|---|
| Diméthicone 1 % | - | stable au moins 15 jours |
| Diméthicone 5 % | instable | stable au moins 15 jours |
| Caprylate/caprate de coprah (mélange 50/50) 1% | - | stable au moins 15 jours |
| Caprylate/caprate de coprah (mélange 50/50) 5% | instable | stable au moins 15 jours |

### EXEMPLE 6

On a testé la stabilité à 40 % de diverses émulsions huile dans eau comportant ou non des concentrations variables d'un agent stabilisant usuel : le Carbomer 941

Les émulsions comportaient toutes 3 % du concentré de l'exemple 1.

La nature et la concentration des huiles, la concentration en Carbomer 941, ainsi que les résultats de stabilité obtenus, figurent dans le Tableau IV ci-dessous.

**Tableau IV**

| | **Carbomer 941** | | | |
|---|---|---|---|---|
| **Emulsion** | 0 % | 0,01 % | 0,05 % | 0,1 % |
| 1 : Huile de paraffine à 5 % | > 7 | - | > 7 | > 7 |
| 2 : Huile de paraffine à 10 % | > 7 | > 7 | > 7 | - |
| 3 : Triheptanoate de glycérol à 5 % | > 7 | - | > 7 | > 7 |
| 4 : Triheptanoate de glycérol à 10 % | > 7 | - | > 7 | > 7 |

Le Symbole "> 7" signifie que l'émulsion est restée stable pendant 7 jours au moins.

On peut constater que toutes les émulsions testées, qu'elles comportent ou non l'agent stabilisant, sont stables pendant au moins 7 jours. Ces résultats montrent qu'une émulsion comportant un concentré selon l'invention ne requiert pas la présence complémentaire d'un agent stabilisant classique.

Les exemples 7 à 13 concernent des compositions cosmétiques ou hygiéniques que l'on peut préparer à l'aide d'un concentré selon l'invention. Dans chacun de ces exemples, le terme "APG" est utilisé pour désigner le concentré de l'exemple 2.

### EXEMPLE 7

### SAVON LIQUIDE

| FORMULE | | |
|---|---|---|
| A | APG | 4.00 % |
| | EAU | 30.00 % |
| B | LESNa | 5.00 % en ingrédient actif |
| | ORAMIX NS10 | 5.00 % en ingrédient actif |
| | EAU | QSP 100% |
| C | ACRYSOL 22 | 2.00 à 3.00 % |
| | EAU | 10.00 % |
| D | PARFUM | QS |
| | CONSERVATEUR | QS |
| | SOUDE | QS pH = 7 |

### MODE OPERATOIRE

Fondre l'APG dans l'eau à environ 60°C (phase A). Laisser refrodir à 30°C puis incorporer la phase B puis la phase C puis la phase D. Enfin ajuster le pH.

### CARACTERISTIQUES

Aspect opaque type émulsion
Viscosité 3000 mPa.s (BROOKFIELD LVT4 6 rpm)

### EXEMPLE 8

### SHAMPOOING A L'HUILE D'AMANDES

| FORMULE | | |
|---|---|---|
| A | APG | 4.00 % |
| | HUILE D'AMANDES DOUCES | 1.00 % |
| | EAU | 30.00 % |
| B | LESNa | 8.00 % en ingrédient actif |
| | ORAMIX NS 10 | 2.00 % en ingrédient actif |
| | AMONYL 675 SB ⁽¹⁾ | 2.00 % en ingrédient actif |
| | EAU | QSP100% |
| C | ACRYSOL 22 | 2.00 à 3.00 % |
| | EAU | 10.00 % |
| D | PARFUM | QS |
| | CONSERVATEUR | QS |
| | SOUDE | QS PH = 7 environ |

| | | |
|---|---|---|
| (1) Cocoamidopropylhydroxysultaïne commercialisée par la Société SEPPIC. | | |

### MODE OPERATOIRE

Fondre l'APG dans l'eau à environ 60°C, incorporer l'huile d'amandes douces (phase A). Laisser refrodiir à 30°C puis incorporer la phase B, la phase C puis la phase D. Enfin ajuster le pH.

### CARACTERISTIQUES

- Aspect nacré type émulsion, stable pendant au moins 3 mois (malgré l'absence d'agent stabilisant).
- Viscosité 300 mPa.s (BROOKFIELD LVT4 6 rpm).

### EXEMPLE 9

### LAIT DEMAQUILLANT

| FORMULE | | |
|---|---|---|
| A | APG | 3.00 % |
| | HUILE DE PARAFFINE EPAISSE | 10.00 % |
| B | EAU | QSP 100 % |
| | CARBOMER 941 | 0.05 % |
| C | PARFUM | QS |
| | CONSERVATEUR | QS |
| | SOUDE | QS PH = 7 environ |

### MODE OPERATOIRE

Fondre A à environ 75°C. Disperser avec un fort cisaillement le Carbomer 941 dans la phase aqueuse et chauffer à 75 °C environ.
Emulsionner la phase B dans la phase A puis, vers 30°C, ajouter la phase C et la quantité de base nécessaire à l'ajustement du pH.

### CARACTERISTIQUES

- Emulsion fluide présentant une texture riche
- temps d'écoulement inférieur à 5 secondes (Norme ISO 2431 - coupe d'écoulement de 6 mm)
- Stabilité supérieure à 3 mois, à la température ambiante.

### EXEMPLE 10

### LAIT CORPOREL

| FORMULE | | |
|---|---|---|
| A | APG | 3.00 % |
| | TRIHEPTANOATE DE GLYCEROL | 10.00 % |
| B | EAU | QSP100 % |
| C | SEPIGEL 305 | 1.00% |
| D | PARFUM | QS |
| | CONSERVATEUR | QS |

### MODE OPERATOIRE

Fondre A à environ 75°C. Chauffer l'eau à 75°C environ.
Emulsionner B dans A. Ajouter C vers 60°C puis, vers 30°C, ajouter D.

### CARACTERISTIQUES

- Emulsion fluide présentant une texture riche
- Stable au moins trois mois à la température ambiante
- Temps d'écoulement inférieur à 5 secondes (Norme ISO 2431 - coupe d'écoulement de 6 mm).

### EXEMPLE 11

### LAIT SOLAIRE

| FORMULE | | |
|---|---|---|
| A | APG | 3.00 % |
| | HUILE DE SESAME | 5.00 % |
| | PARSOL MCX ⁽¹⁾ | 5.000 % |
| | CARRAGHENANE λ | 0.10 % |
| B | EAU | QSP100 % |
| C | SEPIGEL 305 | 0,80% |
| D | PARFUM | QS |
| | CONSERVATEUR | QS |

| | | |
|---|---|---|
| (1) Octylparamethoxycinnamate commercialisé par la société GIVAUDAN. | | |

### MODE OPERATOIRE

Fondre l'APG dans l'huile à environ 75°C. Ajouter le carraghénane et le Parsol. Chauffer l'eau à 75°C environ.
Emulsionner B dans A. Ajouter C vers 60°C puis vers 30°C ajouter D et ajuster le PH si nécessaire.

### CARACTERISTIQUES :

- Emulsion fluide présentant une texture riche.
- Stable au moins 3 mois à la température ambiante.
- Temps d'écoulement inférieur à 5 secondes
   (Norme ISO 2431 - coupe d'écoulement de 6mm)

### EXEMPLE 12

### SHAMPOOING

| FORMULE | | |
|---|---|---|
| A | APG | 4.00 % |
| | ELFACOS GT282S ⁽¹⁾ | 2.00 % |
| | DIMETHICONE 50 mPa.s | 1.00 % |
| | EAU | 30.00 % |
| B | LESNa | 8.00 % |
| | AMONYL 380BA ⁽²⁾ | 5.00 % |
| | EAU | QSP 100% |
| C | JAGUAR C13S ⁽³⁾ | 0,50 % |
| | EAU | 10.00 % |
| D | PARFUM | QS |
| | CONSERVATEUR | QS |

| | | |
|---|---|---|
| (1) Talloweth hydrogénée - 60 myristyl glycol, commercialisée par la société AKZO | | |
| (2) Cocamidopropyl bétaine, commercialisée par la société SEPPIC | | |
| (3) Guar hydroxypropylée, commercialisée par la société RHONE POULENC | | |

### MODE OPERATOIRE :

Fondre l'APG et l'ELFACOS simultanément dans l'eau à environ 60°C et ajouter le DIMETHICONE (phase A). Laisser refroidir à 30°C puis incorporer la phase B puis la phase C puis la phase D. Enfin ajuster le PH à environ 5.0.

### CARACTERISTIQUES :

Aspect opaque type émulsion
Viscosité 1000 mPa.s (BROOKFIELD LVT4 6rpm)

### EXEMPLE 13

### GEL DOUCHE 2 en 1

### (nettoyant et hydratant)

| FORMULE | | |
|---|---|---|
| A | APG | 3,50% |
| | CAPRYLATE/CAPRATE DE COPRAH 50/50 | 4.00% |
| | HUILE DE MONOI | 1.00% |
| | EAU | 30.00% |
| B | LESNa | 12.00% en ingrédient actif |
| | ELFAN OS46A ⁽¹⁾ | 4.00% en ingrédient actif |
| | EAU | QSP 100% |
| C | ACRYSOL 22 | 2 à 3.00% |
| | EAU | 10.00% |
| D | PARFUM | QS |
| | CONSERVATEUR | QS |
| | SOUDE | QS pH = 7 environ |

| | | |
|---|---|---|
| (1) Oléfine (C₁₄ - C₁₆) Sulfonate de sodium, commercialisé par la société AKZO | | |

### MODE OPERATOIRE:

Fondre l'APG dans l'eau à environ 60°C. Incorporer les huiles (phase A). Laisser refroidir à 30°C puis incorporer la phase B puis la phase C puis la phase D. Enfin ajuster le PH.

### CARACTERISTIQUES :

Aspect opaque type émulsion
Viscosité 5000 mPa.s (BROOKFIELD LVT4 6rpm)

## Revendications

1. Concentré comprenant :
i) de 60 à 90 % en poids d'un mélange d'au moins un alkylglycoside de formules (I) et d'au moins un alkylglycoside de formule (II):
RO (G)x (I)
R'O (G')x (II)
dans les quelles :
- R représente un radical aliphatique, linéaire ou ramifié, saturé ou insaturé, comportant de 8 à 15 atomes de carbone.
- R' représente un radical aliphatique linéaire ou ramifié, saturé ou insaturé, comportant de 16 à 22 atomes de carbone.
- G et G', identiques ou différents, représentent un reste de saccharide.
- x et x', identiques ou différents, représentent un nombre compris entre 1 et 10.
ii) de 10 à 40 % en poids d'au moins un alcool gras de formule R₁OH, R₁ étant un radical aliphatique, linéaire ou ramifié, saturé ou insaturé, comportant de 8 à 22 atomes de carbone, et caractérisé en ce que le rapport pondéral entre les alkylglycosides de formule (I) et les alkylglycosides de formule (II) est compris entre 3/1 et 1/3, de préférence compris entre 2/1 et 1/2.

2. Concentré selon la revendication 1 caractérisé en ce que G et G' sont identiques et représentent un reste glucose.

3. Concentré selon l'une des revendications 1 et 2 caractérisé en ce que x et x' représentent un nombre compris entre 1,05 et 2,5, de préférence compris entre 1,05 et 1,35.

4. Concentré selon l'une des revendications 1 à 3 caractérisé en ce qu'il comprend au moins un alkylglycoside de formule (II) dont le radical R' comporte 16 ou 18 atomes de carbone.

5. Concentré selon l'une des revendications 1 à 4 caractérisé en ce qu'il comprend un mélange d'alcools gras, le radical R₁ de chacun de ces alcools gras étant identiques aux radicaux R et R'.

6. Concentré selon la revendication 5 caractérisé en ce que le mélange d'alcools gras comprend au moins 80 %, de préférence de 90 à 99 % en poids d' alcools gras dont le radical R₁ comporte 16 ou plus atomes de carbone.

7. Concentré selon l'une des revendications 1 à 6 caractérisé en ce qu'il comporte de 15 à 38 %, de preférence de 25 à 38 % en poids d'au moins un alcool gras.

8. Concentré selon l'une des revendications 1 à 7 caractérisé en en ce que R, R' et R₁ représentent un radical alkyle, linéaire ou ramifié.

9. Une base nacrante comprenant une dispersion du concentré selon l'une des revendications 1 à 8 dans un liquide tel l'eau.

10. Utilisation d'un concentré selon l'une des revendications 1 à 8 en tant qu'agent nacrant.

11. Utilisation selon la revendication 10 pour la préparation d'une composition moussante choisie dans le groupe constitué par une composition pharmaceutique, cosmétique ou hygiénique, tels un shampooing, un savon liquide, un gel douche, un bain moussant, un gel démaquillant visage ou une composition du type détergent vaisselle ou lessive liquide.

12. Utilisation selon la revendication 11 caractérisée en ce que ladite composition comporte de 2 à 15 % en poids dudit concentré.

13. Utilisation d'un concentré selon l'une des revendications 1 à 8 pour la préparation d'une émulsion du type eau dans huile, huile dans eau ou huile dans eau dans huile.

14. Utilisation selon la revendication 13 caractérisée en ce que ladite émulsion est une émulsion fluide, telle un lait.

15. Utilisation selon l'une des revendications 13 et 14 caractérisée en ce que l'émulsion comporte de 1 à 15 % en poids dudit concentré.

16. Composition comprenant au moins un tensio-actif moussant, une phase huile émulsionnée et un concentré selon l'une des revendications 1 à 8.

17. Composition selon la revendication 16 caractérisée en ce qu'elle consiste en un gel douche 2 en 1.

18. Syndet comprenant un concentré selon l'une des revendications 1 à 8, ainsi qu'un ou plusieurs agents tensioactifs.

19. Syndet selon la revendication 18 caractérisé en ce qu'elle comporte en outre au moins un agent plastifiant, liant ou texturant.

20. Syndet selon l'une des revendications 18 et 19 caractérisé en ce qu'elle comporte 2 à 40 % en poids, de préférence 10 à 35 % en poids dudit concentré, 20 à 50 % en poids d'au moins un tensio-actif moussant et 10 à 40 % en poids d'au moins un agent plastifiant, liant ou texturant.

21. Savon de toilette comprenant un concentré selon l'une des revendications 1 à 8, au moins un savon tensio-actif et, le cas échéant, un agent adoucissant.

## Claims

1. Concentrate comprising:
i) from 60 to 90 % by weight of a mixture of at least one alkylglycoside of formula (I) and of at least one alkylglycoside of formula (II):
RO (G)x (I)
R'O (G')x (II)
in which:
- R represents a saturated or unsaturated linear or branched aliphatic radical containing from 8 to 15 carbon atoms.
- R' represents a saturated or unsaturated linear or branched aliphatic radical containing from 16 to 22 carbon atoms.
- G and G', which are identical or different, represent a saccharide residue.
- x and x', which are identical or different, represent a number between 1 and 10.
ii) from 10 to 40 % by weight of at least one fatty alcohol of formula R₁OH, R₁ being a saturated or unsaturated linear or branched aliphatic radical containing from 8 to 22 carbon atoms, and characterized in that the weight ratio between the alkylglycosides of formula (I) and the alkylglycosides of formula (II) is between 3/1 and 1/3, preferably between 2/1 and 1/2.

2. Concentrate according to Claim 1, characterized in that G and G' are identical and represent a glucose residue.

3. Concentrate according to one of Claims 1 and 2, characterized in that x and x' represent a number between 1.05 and 2.5, preferably between 1.05 and 1.35.

4. Concentrate according to one of Claims 1 to 3, characterized in that it comprises at least one alkylglycoside of formula (II) the radical R' of which contains 16 or 18 carbon atoms.

5. Concentrate according to one of Claims 1 to 4, characterized in that it comprises a mixture of fatty alcohols, the radical R₁ of each of these fatty alcohols being identical to the radicals R and R'.

6. Concentrate according to Claim 5, characterized in that the mixture of fatty alcohols comprises at least 80 %, preferably from 90 to 99 %, by weight of fatty alcohols, the radical R₁ of which contains 16 or more carbon atoms.

7. Concentrate according to one of Claims 1 to 6, characterized in that it contains from 15 to 38 %, preferably from 25 to 38 %, by weight of at least one fatty alcohol.

8. Concentrate according to one of Claims 1 to 7, characterized in that R, R' and R₁ represent a linear or branched alkyl radical.

9. A pearling base comprising a dispersion of the concentrate according to one of Claims 1 to 8 in a liquid such as water.

10. Use of a concentrate according to one of Claims 1 to 8 as a pearling agent.

11. Use according to Claim 10 for the preparation of a foaming composition chosen from the group consisting of a pharmaceutical, cosmetic or hygiene composition, such as a shampoo, a liquid soap, a shower gel, a bubble bath, a makeup-removing gel for the face or a composition of the washing-up detergent or liquid washing product type.

12. Use according to Claim 11, characterized in that the said composition contains from 2 to 15 % by weight of the said concentrate.

13. Use of a concentrate according to one of Claims 1 to 8 for the preparation of an emulsion of the water-in-oil, oil-in-water or oil-in-water-in-oil type.

14. Use according to Claim 13, characterized in that the said emulsion is a fluid emulsion, such as a milk.

15. Use according to one of Claims 13 and 14, characterized in that the emulsion contains from 1 to 15 % by weight of the said concentrate.

16. Composition comprising at least one foaming surfactant, one emulsified oil phase and one concentrate according to one of Claims 1 to 8.

17. Composition according to Claim 16, characterized in that it consists of a 2-in-1 shower gel.

18. Syndet comprising a concentrate according to one of Claims 1 to 8, as well as one or more surface-active agents.

19. Syndet according to Claim 18, characterized in that it also contains at least one plasticizer, binder or texturizing agent.

20. Syndet according to one of Claims 18 and 19, characterized in that it contains 2 to 40 % by weight, preferably 10 to 35 % by weight, of the said concentrate, 20 to 50 % by weight of at least one foaming surfactant and 10 to 40 % by weight of at least one plasticizer, binder or texturizing agent.

21. Cleansing soap comprising a concentrate according to one of Claims 1 to 8, at least one surfactant soap and, where appropriate, a softener.

## Patentansprüche

1. Konzentrat mit:
i) 60 bis 90 Gew.-% einer Mischung aus mindestens einem Alkylglykosid der Formel (I) und mindestens einem Alkylglykosid der Formel (II):
RO (G) x (I)
R'O (G') x (II)
in denen:
- R einen geradkettigen oder verzweigten gesättigten oder ungesättigten aliphatischen Rest mit 8 bis 15 Kohlenstoffatomen darstellt,
- R' einen geradkettigen oder verzweigten gesättigten oder ungesättigten aliphatischen Rest mit 16 bis 22 Kohlenstoffatomen darstellt,
- G und G' gleich oder verschieden sind und einen Saccharidrest darstellen, und
- x und x' gleich oder verschieden sind und eine Zahl zwischen 1 und 10 darstellen,
ii) 10 bis 40 Gew.-% mindestens eines Fettalkohols der Formel R₁OH, wobei R₁ einen geradkettigen oder verzweigten gesättigten oder ungesättigten aliphatischen Rest mit 8 bis 22 Kohlenstoffatomen darstellt, und
dadurch gekennzeichnet, daß das Gewichtsverhältnis zwischen den Alkylglykosiden der Formel (I) und den Alkylglykosiden der Formel (II) zwischen 3/1 und 1/3, vorzugsweise zwischen 2/1 und 1/2, beträgt.

2. Konzentrat nach Anspruch 1, dadurch gekennzeichnet, daß G und G' gleich sind und einen Glucoserest darstellen.

3. Konzentrat nach einem der Ansprüche 1 und 2, dadurch gekennzeichnet, daß x und x' eine Zahl zwischen 1,05 und 2,5, vorzugsweise zwischen 1,05 und 1,35, darstellen.

4. Konzentrat nach einem der Ansprüche 1 bis 3, dadurch gekennzeichnet, daß es mindestens ein Alkylglykosid der Formel (II) enthält, in dem der Rest R' 16 oder 18 Kohlenstoffatome enthält.

5. Konzentrat nach einem der Ansprüche 1 bis 4, dadurch gekennzeichnet, daß es eine Mischung aus Fettalkoholen enthält, wobei der Rest R₁ jedes dieser Fettalkohole gleich den Resten R und R' ist.

6. Konzentrat nach Anspruch 5, dadurch gekennzeichnet, daß die Mischung aus Fettalkoholen zu mindestens 80 Gew.-%, vorzugsweise 90 bis 99 Gew.-%, aus Fettalkoholen besteht, bei denen der Rest R₁ 16 oder mehr Kohlenstoffatome enthält.

7. Konzentrat nach einem der Ansprüche 1 bis 6, dadurch gekennzeichnet, daß es zu 15 bis 38 Gew.-%, vorzugsweise 25 bis 38 Gew.-%, aus mindestens einem Fettalkohol besteht.

8. Konzentrat nach einem der Ansprüche 1 bis 7, dadurch gekennzeichnet, daß R, R' und R₁ einen geradkettigen oder verzweigten Alkylrest darstellen.

9. Perlglanzgrundlage mit einer Dispersion des Konzentrats nach einem der Ansprüche 1 bis 8 in einer Flüssigkeit, wie z.B. Wasser.

10. Verwendung eines Konzentrats nach einem der Ansprüche 1 bis 8 als Perlglanzmittel.

11. Verwendung nach Anspruch 10 zur Herstellung einer schäumenden Zusammensetzung aus der Gruppe der Pharmazeutika, Kosmetika und Hygieneprodukte, wie z.B. eines Shampoos, einer Flüssigseife, eines Duschgels, eines Schaumbads, eines gelförmigen Make-up-Entferners für das Gesicht oder einer Zusammensetzung vom Typ der Geschirrspül- oder Flüssigwaschmittel.

12. Verwendung nach Anspruch 11, dadurch gekennzeichnet, daß diese Zusammensetzung zu 2 bis 15 Gew.-% aus diesem Konzentrat besteht.

13. Verwendung eines Konzentrats nach einem der Ansprüche 1 bis 8 zur Herstellung einer Wasser-in-Öl-, Öl-in-Wasser- oder Öl-in-Wasser-in-Öl-Emulsion.

14. Verwendung nach Anspruch 13, dadurch gekennzeichnet, daß es sich bei dieser Emulsion um eine dünnflüssige Emulsion, wie z.B. eine Milch, handelt.

15. Verwendung nach einem der Ansprüche 13 und 14, dadurch gekennzeichnet, daß die Emulsion zu 1 bis 15 Gew.-% aus diesem Konzentrat besteht.

16. Zusammensetzung mit mindestens einem schäumenden Tensid, einer emulgierten Ölphase und einem Konzentrat nach einem der Ansprüche 1 bis 8.

17. Zusammensetzung nach Anspruch 16, dadurch gekennzeichnet, daß sie aus einem 2-in-1-Duschgel besteht.

18. Syndet mit einem Konzentrat nach einem der Ansprüche 1 bis 8 sowie ein oder mehreren Tensiden.

19. Syndet nach Anspruch 18, dadurch gekennzeichnet, daß es weiterhin mindestens einen Weichmacher, ein Bindemittel oder Texturierungsmittel enthält.

20. Syndet nach einem der Ansprüche 18 und 19, dadurch gekennzeichnet, daß es zu 2 bis 40 Gew.-%, vorzugsweise 10 bis 35 Gew.-%, aus diesem Konzentrat, zu 20 bis 50 Gew.-% aus mindestens einem schäumenden Tensid und zu 10 bis 40 Gew.-% aus mindestens einem Weichmacher, Bindemittel oder Texturierungsmittel besteht.

21. Toiletteseife mit einem Konzentrat nach einem der Ansprüche 1 bis 8, mindestens einer tensidartigen Seife und gegebenenfalls einem Weichmacher.
